(19) Europäisches Patentamt
European Patent Office
Office européen des brevets

(11) Veröffentlichungsnummer: **0 526 793 B1**

(12) # EUROPÄISCHE PATENTSCHRIFT

(45) Veröffentlichungstag der Patentschrift: **28.06.95**

(51) Int. Cl.$^6$: **C07C 209/68**

(21) Anmeldenummer: **92112413.7**

(22) Anmeldetag: **21.07.92**

(54) **Verfahren zur Herstellung von Diphenylaminen.**

(30) Priorität: **03.08.91 DE 4125755**

(43) Veröffentlichungstag der Anmeldung:
**10.02.93 Patentblatt 93/06**

(45) Bekanntmachung des Hinweises auf die Patenterteilung:
**28.06.95 Patentblatt 95/26**

(84) Benannte Vertragsstaaten:
**BE DE FR GB IT**

(56) Entgegenhaltungen:
**EP-A- 0 208 933**
**EP-A- 0 325 132**
**EP-A- 0 494 455**
**DE-A- 2 520 893**

**PATENT ABSTRACTS OF JAPAN vol. 13, no. 39 (C-563)27. Januar 1989**

(73) Patentinhaber: **BAYER AG**
**D-51368 Leverkusen (DE)**

(72) Erfinder: **Immel, Otto, Dr.**
**Immenhofweg 26**
**W-4150 Krefeld (DE)**
Erfinder: **Darsow, Gerhard, Dr.**
**Zu den Tannen 39**
**W-4150 Krefeld (DE)**
Erfinder: **Langer, Reinhard, Dr.**
**Scheiblerstrasse 111**
**W-4150 Krefeld (DE)**
Erfinder: **Buysch, Hans-Josef, Dr.**
**Brandenbuger Strasse 28**
**W-4150 Krefeld (DE)**
Erfinder: **Waldmann, Helmut, Dr.**
**Henry-T.-v.-Böttinger-Strasse 15**
**W-5090 Leverkusen 1 (DE)**

EP 0 526 793 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von Diphenylamin, das durch niederes Alkyl und/oder niederes Alkoxy substituiert sein kann, durch Dehydrierung des korrespondierenden Dicyclohexylamins, des korrespondierendem N-Cyclohexyl-anilins oder eines Gemisches beider in Gegenwart eines Rhodium enthaltenden Trägerkatalysators.

Zur Herstellung von Diphenylamin und dessen Derivaten ist aus DE-OS 23 31 878 ein Verfahren bekannt, bei dem man von Iminen, wie N-Cyclohexyliden-anilin und dessen Derivaten ausgeht und sie in der Gasphase in Gegenwart von Trägerkatalysatoren auf der Basis von Nickel, Platin, Palladium oder Kupfer-Chrom dehydiert.

Weiterhin ist es aus DE-OS 25 20 893 bekannt, Diphenylamin durch katalytische Dehydrierung von Verbindungen und/oder Verbindungsgemischen, die aus ganz oder teilweise hydriertem Diphenylamin bestehen, in Gegenwart eines Nickel-Chrom, Aluminium, Kupfer, Mangan und Alkali enthaltenden Dehydrierungskatalysators herzustellen.

Als solche Verbindungen werden zweikernige aromatische Imine in den Ausführungsbeispielen gezeigt.

In EP 325 132 wird weiterhin ein Verfahren beschrieben, bei dem gegebenenfalls substituiertes Dicyclohexylamin zum korrespondierenden Diphenylamin dehydriert wird, wobei ein Trägerkatalysator eingesetzt wird, der Rhodium und mindestens ein anderes Platinmetall aus der Gruppe Palladium, Platin und Iridium enthält. Dieser Katalysator enthält weiterhin Alkalihydroxid und Alkalisulfat und ist bevorzugt auf einem Träger aus Aluminiumoxid oder Aluminiumspinell aufgebracht, der mit Chrom und Mangan behandelt worden ist. Dieses Verfahren ist ausdrücklich auf eine Rhodium-Platinmetall-Kombination ausgerichtet.

N-Cyclohexylanilin ergibt gemäß Kinet. Katal. 28 (1), 250-254 (zitiert nach C. A. 107 (23), 217 420 z) beispielsweise an Pt (1 %)/$Al_2O_3$ bei 380 °C 41 %. Carbazol und nur 18 % Diphenylamin; Dicyclohexylamin ergibt unter ähnlichen Bedingungen 41 % Carbazol und nur 17 % Diphenylamin. Daneben werden Entaminierungs- und Umlagerungsprodukte, wie Anilin, Benzol, Diphenyl und 4-Amino-diphenyl beobachtet. Angesichts der Kenntnis dieser unerwünschten Nebenreaktionen ist die spezielle Auswahl von Reaktionsbedingungen der genannten EP 325 132 verständlich.

Überraschend war daher die nunmehr gefundene Tatsache, daß ein gegebenenfalls substituiertes Dicyclohexylamin oder ein gegebenenfalls substituiertes N-Cyclohexylanilin entgegen der Lehre von EP 325 132 auch an einem lediglich Rhodium enthaltenden Katalysator glatt zum zugehörigen Diphenylamin umgesetzt werden kann.

Die Erfindung betrifft ein Verfahren zur Herstellung eines Diphenylamins der Formel

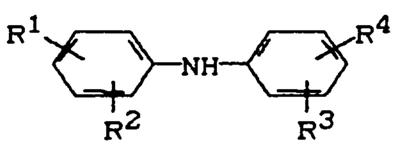 (I),

in der

$R^1$, $R^2$, $R^3$ und $R^4$ unabhängig voneinander Wasserstoff, $C_1$-$C_4$-Alkyl oder $C_1$-$C_4$-Alkoxy bedeuten,

durch katalytische dehydrierende Aromatisierung einer aromatisierbaren Vorstufe eines solchen Diphenylamins unter Anwendung eines Platinmetall-Trägerkatalysators bei 250-450 °C und 0,1-20 bar in der Gasphase, das dadurch gekennzeichnet ist, daß als aromatisierbare Vorstufe ein Dicyclohexylamin oder ein N-Cyclohexyl-anilin der Formeln

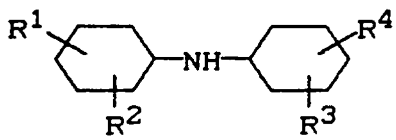 (II), bzw

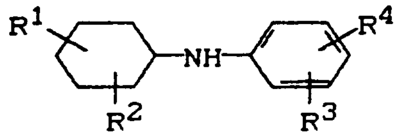 (III) ,

in denen

$R^1$, $R^2$, $R^3$ und $R^4$ die obige Bedeutung haben,

oder ein Gemisch beider eingesetzt wird und als Platinmetall-Katalysator einer angewandt wird, der Rhodium allein auf einem $Al_2O_3$-Träger enthält, wobei der Gehalt an Rhodium 0,05-5 % des Katalysatorgesamtgewichts beträgt, der Katalysator mit einem oder mehreren Promotoren aus der Gruppe der Alkalimetallhydroxide und Alkalimetallsulfate in einer Gesamtmenge von 1 bis 12 % und einer Menge von 1 bis 6 % je einzelner Promotorverbindung, bezogen auf das Katalysatorgesamtgewicht, eingesetzt wird und der Träger einen Gehalt an Chrom und Mangan, gerechnet als Metalle, von zusammen 0,05-8 % des Katalysatorgesamtgewichts mit einem Gewichtsverhältnis von Chrom und Mangan = 5:1-1:5 aufweist.

Das erfindungsgemäße Verfahren wird bei einer Temperatur von 250-450 °C, bevorzugt 300-400 °C und einem Druck von 0,1-20 bar, bevorzugt 0,5-20 bar, besonders bevorzugt 1-6 bar und ganz besonders bevorzugt 1-3 bar in der Gasphase durchgeführt.

Als erfindungsgemäß einzusetzende aromatisierbare Vorstufen des herzustellenden Diphenylamins kommen solche der Formeln (II) bzw. (III) oder ein Gemisch beider in Frage. Im Einsatzmaterial ist ein geringer Anteil von Cyclohexylamin, beispielsweise von bis zu 10 %, bevorzugt bis zu 5 % zulässig, der im Dicyclohexylamin als aromatisierbarer Vorstufe aus der Herstellung dieser Vorstufe enthalten sein kann. N-Cyclohexyl-anilin kann beispielsweise durch selektive Hydrierung aus N-Cyclohexyliden-anilin erhalten werden, es kann aber auch recyclisiertes, nicht vollständig aromatisiertes Material aus der Aromatisierung von Dicyclohexylamin sein.

Beispiele für die Reste $R^1$ bis $R^4$ in den aromatisierbaren Vorstufen sind Wasserstoff, Methyl, Ethyl, Propyl, Isopropyl, Butyl, Isobutyl, Methoxy, Ethoxy, Propoxy, Isopropoxy, Butoxy oder Isobutoxy. In bevorzugter Weise haben die genannten Substituenten die Bedeutung Wasserstoff, $C_1$-$C_2$-Alkyl oder $C_1$-$C_2$-Alkoxy. In bevorzugter Weise haben die genannten Substituenten die Bedeutung Wasserstoff, Methyl oder Methoxy. In weiterhin bevorzugter Weise hat $R^2$ und/oder $R^4$ die Bedeutung Wasserstoff während die Substituenten $R^1$ und/oder $R^3$ neben der Bedeutung Wasserstoff die Bedeutung Alkyl oder Alkoxy im genannten Umfang haben. In ganz besonders bevorzugter Weise richtet sich das Verfahren auf die Herstellung von nicht-substituiertem Diphenylamin aus nicht-substituiertem Dicyclohexylamin oder N-Cyclohexylanilin.

Die umzusetzende aromatisierbare Vorstufe kann als solche allein verdampft und umgesetzt werden. Sie kann weiterhin die oben erwähnten nicht vollständig aromatisierten und recyclisierten Produkte enthalten und kann weiter Begleitstoffe aus der Herstellung der aromatisierbaren Vorstufen enthalten. Des weiteren wird die aromatisierbare Vorstufe bzw. ein Gemisch der aromatisierbaren Vorstufe mit den genannten Begleitstoffen vorteilhaft mit Hilfe eines inerten Trägergasstroms an den Katalysator gebracht. Solche inerten Trägergase sind beispielsweise Stickstoff, Wasserstoff, Argon, niedrige Kohlenwasserstoffe, wie Methan, Ethan oder Erdgas, und andere oder Gemische aus diesen Trägergasen. In bevorzugter Weise werden Stickstoff oder Wasserstoff oder ein Gemisch von ihnen als inertes Trägergas eingesetzt. Das Trägergas wird in einer Menge von 1-100 l/g Ausgangsmaterial, bevorzugt 1-50 l/g Ausgangsmaterial, eingesetzt. Die Katalysatorbelastung wird mit 0,01-1 kg Ausgangsmaterial je Liter Katalysator und Stunde festgesetzt.

Der erfindungsgemäß einsetzbare Katalysator enthält Rhodium auf einem $Al_2O_3$-Träger. Als $Al_2O_3$ kommt $\alpha$-$Al_2O_3$,$\gamma$-$Al_2O_3$, gewöhnlicher Spinell $MgAl_2O_4$, Zinnspinell $ZnAl_2O_4$, oder Eisen- oder Chromspinell in Frage, in denen zweiwertiges Eisen das Magnesium teilweise vertritt oder in denen dreiwertiges Eisen oder Chrom das Aluminium teilweise vertritt. In bevorzugter Weise eignen sich $\alpha$-$Al_2O_3$ und $\gamma$-$Al_2O_3$; in ganz besonders bevorzugter Weise wird $\gamma$-$Al_2O_3$ eingesetzt.

Ein solcher Träger weist einen Gehalt an Chrom und Mangan, gerechnet als Metalle, von zusammen 0,05-8 %, bevorzugt 0,2-5 % des Katalysatorgesamtgewichts auf. Das Gewichtsverhältnis von Chrom und Mangan beträgt 5:1-1:5, bevorzugt 2:1-1:2. Solche mit Chrom und Mangan behandelten Träger sind aus EP 208 933 bekannt.

Der erfindungsgemäß einzusetzende Katalysator enthält Rhodium in einer Gesamtmenge von 0,05-5 %, bevorzugt 0,05-4 %, besonders bevorzugt 0,1-3 % des Katalysatorgesamtgewichts.

Ein solcher Katalysator wird weiterhin mit einem oder mehreren Promotoren aus der Gruppe der Alkalimetallhydroxide und Alkalimetallsulfate eingesetzt. Beispiele für solche Promotoren sind Lithiumhydroxid, Natriumhydroxid, Kaliumhydroxid, Rubidiumhydroxid, Cäsiumhydroxid, Lithiumsulfat, Natriumsulfat, Kaliumsulfat, Rubidiumsulfat, Cäsiumsulfat, bevorzugt Lithiumhydroxid, Natriumhydroxid, Kaliumhydroxid, Lithiumsulfat, Natriumsulfat, Kaliumsulfat, besonders bevorzugt Natriumhydroxid, Kaliumhydroxid, Natriumsulfat und Kaliumsulfat. Diese Promotoren werden in einer Menge von 1-12 %, bevorzugt 2-10 % des Katalysatorgesamtgewichts eingesetzt. Der Gehalt an jeder einzelnen Promotorverbindung ist auf 1-6 %, bevorzugt 2-5 % begrenzt. Hierbei wird in besonders bevorzugter Weise je ein Promotor aus der Gruppe der Alkalimetallhydroxide und aus der Gruppe der Alkalimetallsulfate eingesetzt.

Zur Herstellung der einzusetzenden Katalysatoren kann so vorgegangen werden, daß auf ein $Al_2O_3$ oder ein Spinell in Form von Strangpreßlingen, Pillen oder Kugeln mit Abmessungen von etwa 2-10 mm zunächst Verbindungen des Chroms und Mangans aufgebracht werden. Die Art der Aufbringung solcher Verbindungen durch Tränken oder Besprühen ist dem Fachmann geläufig. Der so beaufschlagte Träger wird auf höhere Temperatur erhitzt (beispielsweise 200-450 °C, bevorzugt 250-350 °C) und anschließend ergänzend mit Rhodium sowie mit den Promotoren beaufschlagt. Nach jedem Auftragen wird eine Trocknung vorgenommen, im allgemeinen bei 100-140 °C bei vermindertem bis normalem Druck, wie 1-1000 mbar, bevorzugt 10-500 mbar, beispielsweise im Wasserstrahlvakuum. Das Aufbringen des Chroms und Mangans auf den Katalysatorträger kann in bevorzugter Art durch gemeinsames Ausfällen eines Mangan-Chrom-Hydroxidgemisches aus einer Chrom- und Mangansalzlösung mit Alkalilauge oder Ammoniak und anschließendes Auswaschen der löslichen Anteile mit Wasser erfolgen. Als Chrom- und Mangansalze kommen insbesondere die Sulfate, Choride, Acetate und/oder Nitrate der genannten Elemente in Betracht. Die Abscheidung des Chroms und Mangans auf dem Katalysatorträger kann auch als Ammonium-Mangan-Chromat oder Ammonium-Alkali-Mangan-Chromat aus einer Lösung von Mangan(II)-Salzen und Ammoniumchromat/-bichromat mittels Ammoniak und/oder basischer Alkaliverbindung erfolgen. Besonders gleichmäßige und festhaftende Abscheidungen erhält man, wenn die Basenzugabe langsam und gleichmäßig unter Vermeidung größerer Konzentrationsunterschiede erfolgt. Hierzu kann beispielsweise die Fällung mittels Harnstoff unter hydrolysierenden Bedingungen vorgenommen werden, wodurch die Bedingungen der langsamen Basezugabe besonders gut gewährleistet sind.

Die Aufbringung der zusätzlichen Bestandteile Rhodium und Promotoren erfolgt, wie bereits beschrieben, nach dem Waschen und Tempern des mit Chrom und Mangan beaufschlagten Trägers. Hierbei kann so vorgegangen werden, daß zunächst das Rhodium, beispielsweise in Form wäßriger Lösungen des Chlorids, Nitrats, Acetats oder anderer geeigneter Salze auf den Träger aufgetränkt werden, wobei nach einer Trocknung eine weitere Tränkung mit einer Alkalihydroxidlösung erfolgen kann. Bei dieser Behandlung wird das Rhodium in Form seines Oxids oder Hydroxids ausgefällt. Der Zusatz der genannten Promotoren erfolgt im allgemeinen nach dem Auswaschen unerwünschter Ionen und einer Trocknung, wobei Alkalimetallhydroxide und/ oder Alkalimetallsulfate gemeinsam oder nacheinander aufgebracht werden. Bei nacheinander durchgeführter Aufbringung wird zwischendurch in der beschriebenen Weise getrocknet. Nach einer abschließenden Trocknung steht der Katalysator grundsätzlich zur Verfügung. In bevorzugter Weise wird er jedoch vor seinem Einsatz durch Behandlung mit Wasserstoff bei erhöhter Temperatur, etwa bei 120-430 °C, bevorzugt bei 150-400 °C aktiviert. Diese Aktivierung kann in besonders bevorzugter Weise nach dem Einfüllen des Katalysators in den Aromatisierungsreaktor erfolgen.

In einer weiteren Variante der Katalysatorherstellung wird der Träger zunächst mit einer Alkalihydroxidlösung getränkt, anschließend getrocknet und anschließend mit einem oder mehreren Rhodiumsalzen beaufschlagt, wobei im Moment der Beaufschlagung auch die Ausfällung von Rhodium in Form des Oxids oder Hydroxids erfolgt. Bei dieser Variante kann das zusätzliche Auftränken eines oder mehrerer Alkalimetallsulfate gemeinsam mit dem Alkalimetallhydroxid oder auch vor oder nach dem Auftragen des Alkalimetallhydroxids erfolgen. Auch hierbei erfolgt nach jedem Auftränken oder Aufsprühen ein separates Trocknen. Auch nach dieser Variante ist der Katalysator grundsätzlich einsatzbereit, kann aber in der beschriebenen Weise vorab mit Wasserstoff bei erhohter Temperatur aktiviert werden.

Geräte zur Herstellung solcher Katalysatoren und die Einstellung der gewünschten Mengen an Wirkstoff durch Wahl der Menge und Konzentration von Tränkungs- oder Sprühlösungen sind dem Fachmann bekannt.

Neben wäßrigen Lösungen der aktiven Elemente auf dem Katalysator kommen grundsätzlich auch alkoholische Lösungen oder Lösungen in niederen Carbonsäuren oder niederen Aminen in Frage, sofern die vorgesehenen Salze der Edelmetalle bzw. die der Promotoren darin löslich sind.

Beispiel 1

50 g eines mit Mangan und Chrom gemäß EP 0 208 933 B1, Beispiel 1, beaufschlagten $\gamma$-$Al_2O_3$ (BET = 350 $m^2/g$) wurden mit einer Lösung getränkt, die aus 1,29 g $RhCl_3$ und 17,5 g Wasser hergestellt worden war. Nach einer Zwischentrocknung wurde der Katalysator 8 Stunden im Wasserstoffstrom reduziert. Anschließend wurde der Katalysator im fließenden Wasser (dest.) chloridfrei gewaschen. Nach einer Zwischentrocknung wurde der Katalysator mit einer Lösung aus 3 g NaOH, 3 g $K_2SO_4$ und 35 g $H_2O$ getränkt und danach 18 Stunden bei 120 °C getrocknet.

20 ml (18,5 g) des so hergestellten rhodiumhaltigen Katalysators wurden in einem senkrecht angeordneten, elektrisch beheizten Glasrohr von 17 mm Innendurchmesser und 70 cm Länge, 71 Stunden im Wasserstoffstrom (30 l/h) bei 400 °C aktiviert. Zur Dehydrierung von Dicyclohexylamin wurde die Ofentemperatur auf 380 °C eingestellt und Stickstoff (10 l/h) als Trägergas verwendet. Dicylohexylamin wurde unter Benutzung einer geeichten Dosiervorrichtung von oben her im Gleichstrom mit dem Trägergas eingeleitet. Im oberen Teil des Reaktionsrohres, in dem sich nur Füllkörper befanden, verdampfte das Dicylohexylamin und strömte zusammen mit dem Trägergas durch die Katalysatorschicht, die sich im mittleren Abschnitt des Reaktionsrohres befand. Das entstandene Reaktionsprodukt wurde auskondensiert und gaschromatographisch analysiert. Unter stationären Reaktionsbedingungen wurden 745 g Dicyclohexylamin innerhalb von 160 Stunden gleichmäßig über den Katalysator geleitet. Das Reaktionsprodukt wurde kondensiert und gaschromatographisch analysiert. Es zeigte folgende Zusammensetzung:

| | |
|---|---|
| Diphenylamin: | 96,7 % |
| Anilin: | 2,2 % |
| Nebenprodukte: | Rest |

Beispiel 2

50 g eines mit Chrom und Mangan gemäß EP-Anmeldung 0 208 933, Beispiel 1, beschichteten $\gamma$-$Al_2O_3$ in Form von Kugeln (etwa 5 mm Durchmesser) und einer BET-Oberfläche von ca. 300 $m^2/g$ wurden mit einer Lösung von 0,66 g $RhCl_3$ in 20 g Wasser gleichmäßig getränkt. Nach einer Zwischentrocknung bei 120 °C erfolgte eine weitere Tränkung mit einer Lösung von 0,73 g NaOH und 1,5 g $K_2SO_4$ in 20 g Wasser. Danach wurde der Katalysator mit dest. Wasser chroridfrei gewaschen, und nach einer Zwischentrocknung wurden 30 g des Katalysators nochmals mit einer Lösung von 0,9 g NaOH und 0,9 g $K_2SO_4$ in 13 g Wasser getränkt und schließlich 16 Stunden im Wasserstrahlvakuum bei 120 °C getrocknet.

30 ml (23,9 g) des so hergestellten Katalysators wurden zunächst 15 h bei 400 °C und dann weitere 20 Stunden bei 380 °C im Wasserstoffstrom (10 l/h) aktiviert. Unter stationären Reaktionsbedingungen wurden bei 380 °C im Verlauf von 69,5 Stunden 436,4 g Dicyclohexylamin über den Katalysator geleitet. Als Trägergas diente Wasserstoff (10 l/h). Das Reaktionsprodukt wurde kondensiert und gaschromatographisch analysiert. Es ergab sich folgende Zusammensetzung:

| | |
|---|---|
| Diphenylamin: | 95,9 % |
| N-Cyclohexylanilin: | 0,4 % |
| Anilin: | 3 % |
| Nebenprodukte: | Rest |

Beispiel 3

40 ml (35,6 g) Rhodiumkatalysator, der gemäß EP 0 208 933 B1, Beispiel 5, hergestellt worden war, wurde in einer Apparatur wie im Beispiel 1 zunächst im Wasserstoffstrom (15 - 20 l/h) bei 400 °C 69 Stunden aktiviert. Dann wurde bei einer Katalysatortemperatur von 373 - 376 °C Dicyclohexylamin unter Benutzung einer geeichten Dosiervorrichtung und Trägergas im Gleichstrom über den Katalysator geleitet.

Der Trägergasstrom bestand aus einem Gemisch von etwa 3 l $H_2$/h und 10 - 13 l $N_2$/h. Bei einer Katalysatorbelastung von 0,22 - 0,34 g Dicyclohexylamin/ml x h zeigte das Reaktionsprodukt folgende Zusammensetzung in Abhängigkeit von den Betriebsstunden des Katalysators (Rest zu 100 % sind Nebenprodukte):

| Betriebsstunden | 71 | 168 | 269 | 349 | 583 | 655 | 849 |
|---|---|---|---|---|---|---|---|
| Diphenylamin | 95,9 | 97,3 | 97,3 | 96,3 | 96,1 | 97,2 | 96 % |
| N-Cyclohexylanilin | 0,3 | - | 0,1 | 0,5 | 0,1 | 0,2 | 0,2 % |
| Anilin | 3,2 | 2,2 | 2,3 | 2,4 | 3,4 | 1,8 | 3,5 % |

Im weiteren Verlauf dieses Versuches wurden die Reaktionsbedingungen variiert, wobei folgende Ergebnisse erzielt wurden:

| Betriebs-stunden | Katalysator-belastung | Reaktions-temperatur | Anilin | N-Cyclohexyl-anilin | Diphenylamin |
|---|---|---|---|---|---|
| h | g/ml·h | °C | % | % | % |
| 1092 | 0,45 | 337 | 3,7 | 0,8 | 94,9 |
| 1187 | 0,74 | 315 | 5,0 | 6,4 | 87,7 |
| 1391 | 0,81 | 318 | 4,1 | 7,3 | 87,6 |
| 1561 | 0,72 | 310 | 5,4 | 11,1 | 82,3 |
| 1636 | 0,72 | 330 | 3,6 | 6,0 | 89,4 |
| 1758 | 0,44 | 368 | 10,2 | 0,1 | 88,7 |
| 1802 | 0,44 | 368 | 3,3 | 0,5 | 96,6 |

Beispiel 4

20 ml (18,1 g) des gleichen Rhodiumkatalysators wie in Beispiel 3 wurde in einer Apparatur wie in Beispiel 1 zunächst im Wasserstoffstrom (30 l/h) 64 Stunden bei 400 °C aktiviert. Zur Dehydrierung von N-Cyclohexylanilin wurde die Ofentemperatur auf 410 °C eingestellt und Stickstoff (5 l/h) als Trägergas verwendet. Im Verlauf von 5 Stunden wurden 16,3 g N-Cyclohexyl-anilin durch die Katalysatorschicht

geleitet. Das Reaktionsprodukt wurde kondensiert und gaschromatographisch analysiert. Es ergab sich folgende Zusammensetzung:

| | |
|---|---|
| Diphenylamin: | 90,1 % |
| N-Cyclohexylanilin: | 0,2 % |
| Anilin: | 6,5 % |
| Nebenprodukte: | Rest |

## Patentansprüche

1. Verfahren zur Herstellung eines Diphenylamins der Formel

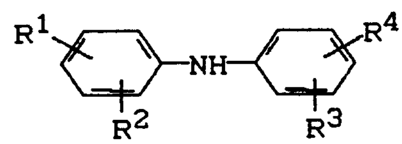

,

in der

$R^1$, $R^2$, $R^3$ und $R^4$ unabhängig voneinander Wasserstoff, $C_1$-$C_4$-Alkyl oder $C_1$-$C_4$-Alkoxy bedeuten,

durch katalytische dehydrierende Aromatisierung einer aromatisierbaren Vorstufe eines solchen Diphenylamins unter Anwendung eines Platinmetall-Trägerkatalysators bei 250-450 °C und 0,1-20 bar in der Gasphase, dadurch gekennzeichnet, daß als aromatisierbare Vorstufe ein Dicyclohexylamin oder ein N-Cyclohexyl-anilin der Formeln

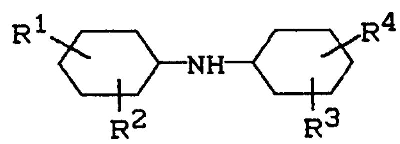

bzw.

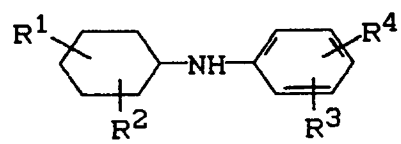

,

in denen

$R^1$, $R^2$, $R^3$ und $R^4$ die obige Bedeutung haben,

oder ein Gemisch beider eingesetzt wird und als Platinmetall-Katalysator einer angewandt wird, der Rhodium allein auf einem $Al_2O_3$-Träger enthält, wobei der Gehalt an Rhodium 0,05-5 % des Katalysatorgesamtgewichts beträgt, der Katalysator mit einem oder mehreren Promotoren aus der Gruppe der Alkalimetallhydroxide und Alkalimetallsulfate in einer Gesamtmenge von 1 bis 12 % und einer Menge von 1 bis 6 % je einzelner Promotorverbindung, bezogen auf das Katalysatorgesamtgewicht, eingesetzt wird und der Träger einen Gehalt an Chrom und Mangan, gerechnet als Metalle, von zusammen 0,05 bis 8 % des Katalysatorgesamtgewichts mit einem Gewichtsverhältnis Cr:Mn = 5:1 bis 1:5 aufweist.

2. Verfahren nach Anspruch 1, dadurch gekennzeichnet, daß der Gehalt an Rhodium 0,05-4 %, bevorzugt 0,1-3 % des Katalysatorgewichts beträgt.

3. Verfahren nach Anspruch 1, dadurch gekennzeichnet, daß Chrom und Mangan zusammen 0,2-5 % des Gesamtgewichts des Katalysators darstellen.

4. Verfahren nach Anspruch 1, dadurch gekennzeichnet, daß das Gewichtsverhältnis von Chrom und Mangan 2:1-1:2 beträgt.

5. Verfahren nach Anspruch 1, dadurch gekennzeichnet, daß der Katalysator als Promotoren 2-10 % des Katalysatorgesamtgewichts an einem oder mehreren Alkalihydroxid(en) und/oder Alkalimetallsulfat(en) enthält, wobei der Gehalt an jeder einzelnen Promotorverbindung 2-5 % begrenzt ist.

6. Verfahren nach Anspruch 1, dadurch gekennzeichnet, daß ein oder mehrere Promotoren aus der Gruppe Lithiumhydroxid, Natriumhydroxid, Kaliumhydroxid, Lithiumsulfat, Natriumsulfat und Kaliumsulfat eingesetzt wird (werden).

7. Verfahren nach Anspruch 1, dadurch gekennzeichnet, daß der mit Chrom und Mangan beaufschlagte Träger vor dem Aufbringen von Rhodium auf 200-450 °C, bevorzugt 250-350 °C erhitzt wird.

8. Verfahren nach Anspruch 1, dadurch gekennzeichnet, daß nach dem Aufbringen von Rhodium eine Aktivierung durch Behandlung mit Wasserstoff bei 120 bis 450 °C, bevorzugt bei 200 bis 420 °C erfolgt.

9. Verfahren nach Anspruch 1, dadurch gekennzeichnet, daß je ein Promotor aus der Gruppe der Alkalimetallhydroxide und aus der Gruppe der Alkalimetallsulfate eingesetzt wird.

## Claims

1. Process for the preparation of a diphenylamine of the formula

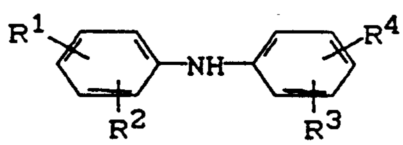

in which

$R^1$, $R^2$, $R^3$ and $R^4$, independently of one another, denote hydrogen, $C_1$-$C_4$-alkyl or $C_1$-$C_4$-alkoxy,

by catalytic dehydrogenating aromatisation of an aromatisable precursor of such a diphenylamine, using a supported platinum metal catalyst at 250-450 °C and 0.1-20 bar in the gas phase, characterised in that the aromatisable precursor used is a dicyclohexylamine or an N-cyclohexyl-aniline of the formula

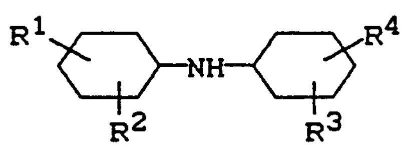

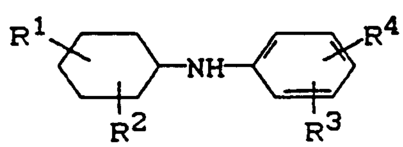

in which

$R^1$, $R^2$, $R^3$ and $R^4$ have the above meaning,

or a mixture of both, and the platinum metal catalyst used is one containing rhodium alone on an $Al_2O_3$ support, the rhodium content being 0.05-5 % of the total weight of the catalyst, the catalyst being used with one or more promoters from the group of the alkali metal hydroxides and alkali metal sulphates in a total amount of 1 to 12% and an amount of 1 to 6% for each individual promotor compound, relative to the total weight of the catalyst, and the support having a chromium and manganese content, calculated as metals, of together 0.05 to 8% of the total weight of the catalyst, with a weight ratio Cr:MA of 5:1 to 1:5.

2. Process according to Claim 1, characterised in that the rhodium content is 0.05-4 %, preferably 0.1-3 %, of the catalyst weight.

3. Process according to Claim 1, characterised in that chromium and manganese together represent 0.2-5 % of the total weight of the catalyst.

4. Process according to Claim 1, characterised in that the weight ratio of chromium to manganese is 2:1-1:2.

5. Process according to Claim 1, characterised in that the catalyst contains, as promoters, 2-10 % of one or more alkali metal hydroxide(s) and/or alkali metal sulphate(s), relative to the total weight of the catalyst, the level of each individual promoter compound being limited to 2-5 %.

6. Process according to Claim 1, characterised in that one or more promoters from the group consisting of lithium hydroxide, sodium hydroxide, potassium hydroxide, lithium sulphate, sodium sulphate and potassium sulphate is (are) used.

7. Process according to Claim 1, characterised in that the support loaded with chromium and manganese is heated to 200-450 ° C, preferably 250-350 ° C, before the rhodium is applied.

8. Process according to Claim 1, characterised in that after the rhodium has been applied, activation by means of a treatment with hydrogen at 120 to 450 ° C, preferably at 200 to 420 ° C, takes place.

9. Process according to Claim 1, characterised in that one promoter each from the group of the alkali metal hydroxides and from the group of the alkali metal sulphates is used.

**Revendications**

1. Procédé de préparation d'une diphénylamine de formule :

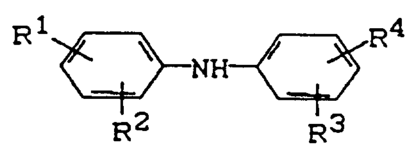

dans laquelle
$R^1$, $R^2$, $R^3$ et $R^4$ représentent chacun, indépendamment les uns des autres, l'hydrogène, un groupe alkyle en $C_1$ à $C_4$ ou alcoxy en $C_1$ à $C_4$,
par aromatisation déshydrogénante catalytique d'un produit précurseur aromatisable d'une telle diphénylamine avec utilisation d'un catalyseur consistant en un métal de la série du platine sur support, à des températures de 250 à 450 ° C et des pressions de 0,1 à 20 bar en phase gazeuse, caractérisé en ce que l'on met en oeuvre en tant que produit précurseur aromatisable une dicyclohexylamine ou une N-cyclohexylaniline de formules respectives :

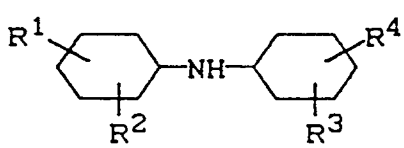

et

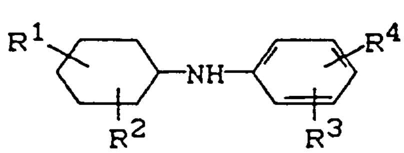

dans lesquelles
$R^1$, $R^2$, $R^3$ et $R^4$ ont les significations indiquées ci-dessus,
ou un mélange de ces deux produits précurseurs, et on utilise en tant que catalyseur à base d'un métal de la série du platine, un catalyseur qui contient uniquement du rhodium sur un support d'$Al_2O_3$, la teneur en rhodium représentant de 0,05 à 5 % du poids total du catalyseur, on met en oeuvre le catalyseur avec un ou plusieurs activateurs pris dans le groupe formé par les hydroxydes de métaux alcalins et les sulfates de métaux alcalins en quantité totale de 1 à 12 % et en quantité de 1 à 6 % de chacun des activateurs, par rapport au poids total du catalyseur, et le support contient du chrome et du manganèse en proportions totales, exprimées en métaux, de 0,05 à 8 % du poids total du catalyseur, avec un rapport en poids Cr/Mn de 5:1 à 1:5.

**2.** Procédé selon la revendication 1, caractérisé en ce que la teneur en rhodium représente de 0,05 à 4 % en poids, de préférence de 0,1 à 3 % du poids du catalyseur.

**3.** Procédé selon la revendication 1, caractérisé en ce que le chrome et le manganèse représentent au total 0,2 à 5 % du poids total du catalyseur.

**4.** Procédé selon la revendication 1, caractérisé en ce que les proportions relatives en poids entre le chrome et le manganèse vont de 2:1 à 1:2.

**5.** Procédé selon la revendication 1, caractérisé en ce que le catalyseur contient en tant qu'activateurs, en proportions de 2 à 10 % de son poids total, un ou plusieurs hydroxydes alcalins et/ou sulfates de métaux alcalins, la teneur en chacun des activateurs individuels étant limitée à un niveau de 2 à 5 %.

**6.** Procédé selon la revendication 1, caractérisé en ce que l'on utilise un ou plusieurs activateurs pris dans le groupe formé par l'hydroxyde de lithium, l'hydroxyde de sodium, l'hydroxyde de potassium, le sulfate de lithium, le sulfate de sodium et le sulfate de potassium.

**7.** Procédé selon la revendication 1, caractérisé en ce que, avant application du rhodium, on chauffe le support imprégné de chrome et de manganèse à des températures de 200 à 450 °C, de préférence de 250 à 350 °C.

**8.** Procédé selon la revendication 1, caractérisé en ce que, après application du rhodium, on soumet le catalyseur à activation par traitement à l'aide d'hydrogène à des températures de 120 à 450 °C, de préférence de 200 à 420 °C.

**9.** Procédé selon la revendication 1, caractérisé en ce que l'on utilise un activateur pris parmi les hydroxydes de métaux alcalins et un activateurs pris parmi les sulfates de métaux alcalins.